# EUROPEAN PATENT APPLICATION

(11) **EP 4 066 677 A1**
(43) Date of publication of application: **05.10.2022**
(21) Application number: 21166415.6
(22) Date of filing: 31.03.2021
(51) Int. Cl.: A44B 18/00, D04H 11/08, A61F 13/62, A61F 13/56

(54) **RECLOSABLE FASTENER WEB, METHOD FOR MAKING THE SAME, FIBROUS HOOK AND LOOP CLOSURE SYSTEM, ELASTIC EAR AND HYGIENE ARTICLE**

(71) Applicant: 3M Innovative Properties Company, Saint Paul, MN 55133-3427 (US)
(72) Inventor: Hertlein, Thomas, 41352 Korschenbroich (DE)
(74) Representative: Vollmers, Hans-Gerd

(57) **Abstract**

The present disclosure relates to a reclosable fastener web 100, 100' having a backing layer 20, 20' and U-shaped fasteners 40, 40' comprising two fastening elements 40A, 40B each having a stem 36 and an enlarged portion 38 at the distal end. The stem 36 is a section of a fiber with an elongation at break of less than 400 % and the stem 36 has a linear density of less than 50 dtex. The present disclosure further relates to a method of making a reclosable fastener web 100, 100'. Moreover, the present disclosure relates to a fibrous hook and loop component comprising a reclosable fastener web 100, 100' according to the present disclosure as well as to an elastic ear 810, 810' for a hygiene article 820, 820' and to a hygiene article 820, 820' comprising a reclosable fastener web 100, 100' according to the present disclosure. The reclosable fastener web 100, 100' according to the present disclosure provides a soft touch and textile appearance of a fastener system and reduces skin irritations thereby. Also, a cost-effective fastening system is thereby provided.

## Description

### Technical field

The present disclosure relates to a reclosable fastener web for engaging and to a method for making a reclosable fastener web. The present disclosure further relates to a fibrous hook and loop closure system, to a hygiene article and to an elastic ear with a reclosable fastener web according to the present disclosure.

### Background

Releasable fastener or closure systems, also called hook and loop fasteners, include a plurality of closely spaced upstanding projections with loop-engaging heads, also called hook members. The hook members can releasably engage with woven, nonwoven, or knitted loops, the corresponding loop members. Mechanical fastener systems are used to provide releasable attachments in numerous applications, in particular fastener systems for disposable absorbent articles like diapers, napkins etc. Hook structures of various shapes can be made from thermoplastic materials by molding, for example as described in U. S. Patent No. 3,594,865. Alternatively, hook members may be formed from precursors. The precursors are also formed by molding of a thermoplastic resin yielding a web containing a plurality of precursors on a backing. The web is then subjected to a capping step where the precursors are shaped into hooks, resulting in a plurality of fasteners on the backing. Such methods are described, for example, in international patent application Nos. WO 94/23610 and 92/04839. Other technologies for making mechanical fasteners are described, for example, in US 7,198,743 B2 or in EP-B-258 22 61.

While the existing methods for making reclosable fastener web with mechanical fasteners represent established and reliable processes, the manufacture of the mechanical fasteners may be cost-intensive. Some of the existing solutions may also be of a certain stiffness with relative rigid fastening elements which may cause skin irritation when contacting the wearer's skin. Some of the existing solutions may also have a solid plastic appearance that does not fit with the textile appearance of the remaining disposable absorbent article.

Therefore, a need exists to provide a reclosable fastener web which can be manufactured in a cost-efficient way and which provides for a soft touch and textile appearance with comparably fine stems.

### Summary

In one aspect, the present disclosure relates to a reclosable fastener web comprising a backing layer having a first and a second major surface and a plurality of U-shaped fasteners attached to the first major surface of the backing layer. The U-shaped fasteners each comprise two fastening elements each having a stem with an enlarged portion at the distal ends. The U-shaped fasteners further comprise a flat intermediate portion between the two stems. The stem is a section of a fiber having an elongation at break of less than 400 %, preferably of less than 350 %. The stem has a linear density of less than 50 dtex. Such a stem, e. g. made of polypropylene, with a linear density of e. g. 50 dtex, i. e. a weight of 50g/10000m fiber length, has a cross-sectional area of about 5555 µm² given a density of polypropylene of about 0,9 g/m³. In case of a round cross section of such a stem the diameter of the stem would be about 84 µm. The advantage of such a reclosable fastener web is that a cost-efficient way of manufacturing a reclosable fastener web with mechanical fastening elements is provided. Also, the reclosable fastener has a soft touch and textile appearance with comparably fine stems and reduces or avoids irritations to the wearer's skin even when contacting the skin.

A reclosable fastener web within the meaning of the present disclosure is understood as a web with upstanding mechanical fastening elements for engagement of corresponding loop or fibrous structures. The mechanical fastening elements or so-called hooks typically comprise enlarged portions at their distal ends. The mechanical fastening elements may also be capable of engaging with themselves and with other surfaces e. g. the backing, of the reclosable fastener web.

A backing layer within the meaning of the present disclosure is understood as a base layer for carrying mechanical fastening elements attached thereto. The backing layer may comprise or consist of a film layer, a fibrous structure such as a nonwoven or combinations thereof. The backing layer may comprise more than one layer. Typically, the backing layer has a first and a second major surface. The first and the second major surface may be made of the same kind and structure or may be different. Typically, the backing layer has a basis weight in the range of from 10 to 100 g/m² as measured according to ISO 536.

Fastening elements within the meaning of the present disclosure are understood as substantially upstanding elements protruding from a backing layer. The fasteners of the present disclosure are U-shaped and comprise two fastening elements each having a stem and a flat intermediate portion between the stems and connected therewith. Typically, the fasteners or fastening elements are made from a polymeric material, preferably from a spun polypropylene fiber. Typically, fasteners or fastening elements comprise enlarged portions at their distal ends, i. e. the ends opposite to where the stems of the fasting elements are connected to a backing layer. These enlarged portions are also referred to as caps. A suitable process for providing the caps at the fastening elements is referred to as capping and preferably utilizes radiant heat applied to the ends of the stems thereby deforming the ends into the enlarged portions or caps. Capping of fastening elements is described, for example, in US 4,454,183 (Sandford) or US 9,872,542 (Barker et al.). Other methods are conceivable, although not preferred, as e. g. described in US 5,077,870 (Melbye et al.) or EP-B-2 582 261 (Wood et. al.). Fastening elements are arranged on the backing with a certain distance to each other, which is also referred to as spacing. The spacing may be regular or irregular, the latter of which is considered as random spacing. The spacing may be the same in length and width direction of the backing layer from which the fastening elements protrude. It is also conceivable that the spacing is regular in one direction, e. g. the length direction of the backing layer, whereas the spacing in the other direction, e. g. the width direction of the backing, is a random spacing. It is further conceivable that the spacing is regular or irregular only along a certain distance of the length or width extension of the backing layer, so that a combination of regular and irregular spacing may be found over the length and/or width extension of the backing. The stems of the fastening elements typically have a circular cross-section. It is also conceivable that the stems have a non-circular cross-section, preferably the cross-section has one or more lobes.

Fibers within the meaning of the present disclosure are understood as elongated elements. The fibers typically have a fineness or linear density measured in dtex (1 dtex = 1 gram/10000 m fiber length). Fibers typically have an elongation at break measured in percent. The elongation at break may be measured according to ISO 5079. Typical materials suitable for the fibers include polypropylene homopolymer, polypropylene copolymer and potentially bicomponent fibers comprising polypropylene. Fibers typically are provided as fibrous web, having an endless length and a width corresponding to the production width of a reclosable fastener web. The fibrous web can comprise endless mono- or multi-filaments, a pre-bonded nonwoven webs comprising endless filament fibers or cut staple fibers, or an unbonded nonwoven. Fibers may be provided in an endless form and are typically wound up onto rolls or spools. During manufacture, the fibers are unwound from the roll or spool and typically are further processed, e. g. converted into fibrous webs or nonwovens. It is noted that the terms fiber web and fibrous web are used synonymously throughout the description. In another aspect, the present disclosure also relates to a method for making a reclosable fastener web. The method comprises the steps of providing a fiber web comprising fibers with a linear density of less than 50 dtex; preferably of less than 30 dtex, most preferred of less than 20 dtex; raising the fibers of the fiber web to provide fiber loops; increase the number of fiber end sections by cutting fibers of the raised fiber loops to provide stems; providing enlarged portions at the distal ends of the stems. The stems preferably have a non-circular cross section with at least one lobe. The advantage of such a method is that a cost-efficient and reliable way of making fastening elements is provided. The resulting reclosable fastener web with its fastening elements has a soft touch and textile appearance with comparably fine stems reducing or avoiding skin irritations even when in contact with the wearer's skin. The very soft fastener also allows a large hook area, resulting in a reliable closure. It is understood that the steps "increase the number of fiber end sections by cutting fibers of the raised fiber loops to provide stems" and "providing enlarged portions at the distal ends of the stems" may be performed in one step during the manufacturing.

In a further aspect, the present disclosure furthermore relates to a fibrous hook and loop closure system comprising a loop component. The loop component comprises a loop surface comprising loops which are sections of a fiber and which are configured and arranged for engaging with a hook component. The fibrous hook and loop closure system comprises a hook component comprising a reclosable fastener web according to the present disclosure comprising U-shaped fasteners for engaging with the loops of the loop component. The U-shaped fasteners each comprising two fastening elements each having a stem which is a section of a fiber with a linear density of less than 50 dtex and which comprise enlarged portion at their distal ends. The ratio of the linear density of the fibers from which the stems are made divided by the linear density of the fibers from which the loops are made is less than 12, preferably less than 7. The fastening elements are made from fibers preferably having a non-circular cross section with at least one lobe. The advantage of such a fibrous hook and loop closure system is that a cost-efficient and reliable fastening system is provided thereby. The hook component has a soft touch and textile appearance with comparably fine stems which reduces or avoids skin irritations even when in contact with the wearer's skin.

In yet a further aspect, the present disclosure moreover relates to an elastic ear. Elastic ears are typically used in hygiene articles, e. g. disposable absorbent articles such as diapers and typically have a hook tab attached at the distal end of the ear. The elastic ear of the present disclosure comprises an elastic center layer, at least one nonwoven skin layer and a reclosable fastener web according to the present disclosure attached to the hygiene article-facing side of the elastic ear. The outer edge of the reclosable fastener web preferably does not exceed the outer edges of the elastic ear. The elastic ear material may need to be activated, e. g. for skin layers which do not exhibit a large elongation. For example, such activation may be done by feeding the material through a nip of intermeshing rollers that stretch the ear in cross direction. This process is also being referred to as ring-rolling. Other methods are conceivable such as stretching by diverging disks. The advantage of such an elastic ear is that the strength of the ear does allow a backing layer for the reclosable fastener web with very light basis weight, e. g. a 17 g/m² spunbond nonwoven. This results in a cost-efficient diaper closure. The backing layer could be made of a nonwoven that allows for ring rolling, resulting in an elasticated reclosable fastener web. The reclosable fastener web having a low basis weight backing layer has a soft touch and textile appearance with comparably fine stems which reduces or avoids skin irritations even when in contact with the wearer's skin.

In still another aspect, the present disclosure also relates to a hygiene article comprising a reclosable fastener web according to the present disclosure. The advantage of the hygiene article is that it is provided in a cost-efficient and reliable way thereby. The reclosable fastener web has a soft touch and textile appearance with comparably fine stems which reduces or avoids skin irritations even when in contact with the wearer's skin or when applied in larger areas. Such disposable article may include disposable absorbent articles such as diapers, sanitary napkins, sanitary or medical pads or incontinence briefs or pads. In such articles the reclosable fastener web may engage to dedicated loop landing zones, backsheet or topsheet nonwovens, or knitted or woven fabrics, e. g. cotton underwear.

In one embodiment, the reclosable fastener web comprises repeatedly arranged bonding areas at which the stems are attached with their flat intermediate portions to the first major surface of the backing layer. The repeat pattern may be provided by a tool that is used to form a flat fiber web into a corrugated fiber web. The bonding areas may be formed by ultrasonic bonding fibers from which the stems are made to the backing or by entirely or partially embedding fibers from which the stems are made into a molten film which at a later process stage provides the backing layer. The advantage of such bonding areas is that a reliable way is provided for bonding the fastening elements to the backing layer without use of extra adhesive.

In another embodiment, the plurality of U-shaped fasteners of the reclosable fastener web protrude from the first major surface of the backing layer with random spacing along the bonding area. The random spacing is preferably present in one direction or extension, e. g. the length or width direction, of the backing layer only. However, it is also conceivable that the random spacing is also present in the other direction or extension, i. e. the length and the width, of the backing layer. The advantage of such a random spacing is that it allows to make reclosable fastener webs with a high hook density without the need to align e. g. individual monofilaments.

In a further embodiment, the reclosable fastener web is self-engaging. Self-engaging may include that the fastening elements engage with each other and/or that the fastening elements engage with the backing layer of the reclosable fastener web, either at the major surface from which the fastening elements protrude or at the opposite major surface, or both major surfaces. The advantage of such a self-engaging reclosable fastener web is that if the reclosable fastener web is used as closure tab, the fastener tab can be folded onto itself and stay closed until the end user opens the tab again. This so-called antiflagging feature allows for reliable processing on a diaper line and provides a defined release surface for the fastener web. The risk of a too high or too low engagement to the nonwoven skin layer of an elastic ear is eliminated.

In yet another embodiment, the stems of the reclosable fastener web have a linear density of less than 30 dtex, preferably of less than 20 dtex, most preferred less than 15 dtex. The advantage of such stems is that a soft touch and textile appearance with comparably fine stems of the reclosable fastener web is achieved thereby leading to less or no skin irritations of the wearer when in contact therewith. The low linear density and the related low bending stiffness also improves the processing step of forming a flat fiber web into arcuate portions. Thinner fibers also provide higher hook density and improved coverage.

In still another embodiment, the stems of the reclosable fastener web have a non-circular cross section having at least one lobe. The advantage of such non-circular stems with at least one lobe is that a better closure performance is achieved for enlarged portions of the stems of the U-shaped fasteners, so-called caps, formed from such fibers (e. g. palm-tree shaped caps) versus other shapes, e. g. resulting from circular cross-sections of fibers and stems, respectively, so-called mushroom shaped caps.

In one embodiment of the method of making a reclosable fastener web, the step of providing fibers comprises the steps of providing a plurality of fibers; making a fiber web from the provided fibers and optionally bonding the fiber web. The fiber web may be made from a plurality of mono- or multi-filaments that are provided from a plurality of spools and aligned substantially parallel and with uniform spacing between the fibers to form a fiber web. Preferably a plurality of such filaments can also be wound on a beam, so that a fiber web of aligned filaments with a given spacing can be unwound. Another preferred option for the fiber web is a bonded nonwoven that is unwound like a beam with filaments. The bonding of the fiber web may include thermal bonding such as calander bonding or air through bonding, or mechanical bonding such as needle punching. The most preferred option for the fiber web is an unbonded nonwoven web, e. g. carded web. The advantage of such a method with a web making step is that it eliminates handling of a high number of spools. This allows a production at large width. The use of an unbonded, e. g. carded web, allows very fast change in hook densities, just by changing the basis weight of the fiber web.

In another embodiment of the method of making a reclosable fastener web, the step of forming raised fiber loops includes the steps of forming arcuate portions of the fiber web; providing a backing layer and fixing the arcuate portions to the backing layer to create fiber loops. The arcuate portions may be formed by a pair of intermeshing rollers with a corrugated surface, wherein the fiber web is run through a nip formed by these corrugation rollers such that arcuate portions are formed thereby. These arcuate portions may have a shape of a sinusoidal wave or a zig-zag shape or combinations thereof. The step of providing a backing layer may include unwinding a backing layer from a supply roll or providing the backing layer as molten film, that after solidification forms a solid backing layer. The backing layer is fed to the fiber web with the arcuate portions formed thereon. The step of fixing the arcuate portion to the backing layer to create fiber loops may include thermal bonding, ultrasonic bonding, or embedding the fiber web into a molten film layer. The arcuate portions may have portions that are substantially parallel to the backing layer the arcuate portions are fixed to. The advantage of such a method with the formation of arcuate portions of the fiber web is that fiber loops are provided in a reliable way. To produce loop landing zones such method is used for many years. Reliable equipment exists that allows robust processing of such loops.

In a further embodiment of the method of making a reclosable fastener web by fixing the arcuate portions onto the backing layer, flat intermediate portions are formed on the fiber web at which the fiber loops are bonded to the backing layer. The advantage of such a method with forming flat intermediate portions is that a reliable way is provided of connecting and attaching fibers to the backing layer from which stems and fastening elements are made in a later processing step.

In yet a further embodiment of the method, the method comprises the steps of compressing the reclosable fastener web from an initial thickness T to a compressed thickness t and winding the reclosable fastener web in the compressed state. The compressed thickness t is less than 0,8 T, preferably less than 0,6 T. The initial thickness and/or the compressed thickness may be measured by a microscope, e. g. Keyence VHX-2000. The compressed thickness of a fastener web in a roll might be calculated by the formula: t = (Dᵣₒ² - D_{co}²)*π/(4*I), wherein Dᵣₒ is the outer roll diameter, D_{co} is the outer core diameter and I is the length of the wound fastener web. The advantage of such a method with compressing the reclosable fastener web is that the thickness of the web on a roll, onto which the reclosable fastener web is typically wound, is lower and thus, a higher roll length of the wound up web is achieved thereby. This may be beneficial as the roll needs to be replaced with a lower frequency leading to a more efficient manufacturing process.

In one embodiment, the reclosable fastener web comprises a plurality of mechanical fastener tabs provided on the reclosable fastener web side-by-side in an endless form, wherein individual mechanical fastener tabs can be cut from the reclosable fastener web. The advantage of such an arrangement is that a reliable and efficient manufacturing is achieved thereby. Lamination of hook stripes to a carrier is eliminated as the entire tab surface may have fasteners resulting in a reliable closure.

The invention was described in various embodiments above. It is understood by a person skilled in the art that one of, several of or all the above-mentioned embodiments can be combined with each other.

The invention will now be described in more detail with reference to the following Figures exemplifying particular embodiments of the invention:

### Brief description of the Figures

Fig. 1 shows in a schematic perspective view a precursor of the reclosable fastener web according to an embodiment of the present disclosure;
Fig. 2 shows in a schematic perspective view a later stage of the precursor of the reclosable fastener web as shown in Fig. 1;
Fig. 3 shows in a schematic perspective view the reclosable fastener web according to an embodiment of the present disclosure as obtained from the precursors as shown in Figs. 1 and 2;
Fig. 4 shows in a schematic functional view an apparatus for and a method of making a reclosable fastener web according to an embodiment of the present disclosure;
Fig. 5 shows in a schematic functional view an apparatus for and a further method of making a reclosable fastener web according to an embodiment of the present disclosure;
Fig. 6 shows in a schematic functional view a method of making a precursor of the reclosable fastener web according to an embodiment of the present disclosure;
Fig. 7 shows in a schematic functional view a further method of making a precursor of the reclosable fastener web according to an embodiment of the present disclosure;
Fig. 8 shows in a schematic functional view another method of making a precursor of the reclosable fastener web according to an embodiment of the present disclosure;
Fig. 9 shows in a schematic functional view still another method of making a precursor of the reclosable fastener web according to an embodiment of the present disclosure;
Fig. 10 shows in a schematic functional view the method of making a precursor as shown in

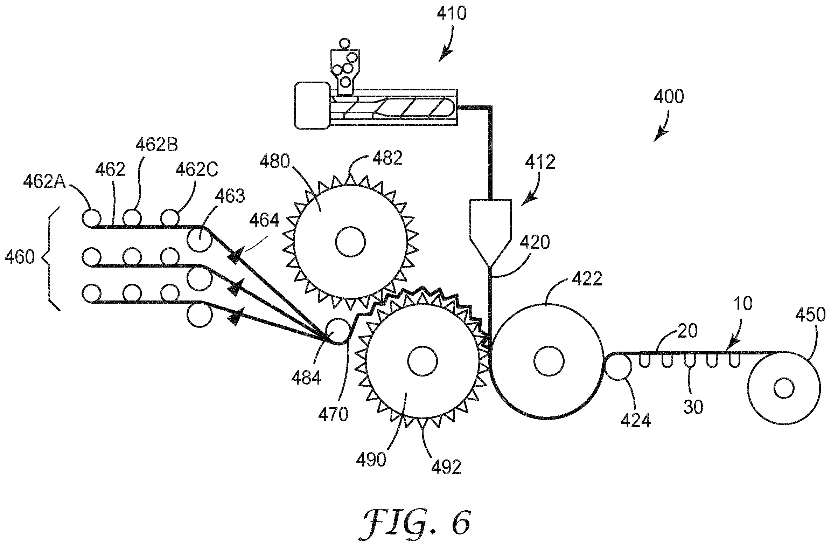

Fig. 6 with a different step of providing a backing and of bonding the fiber web to the backing;

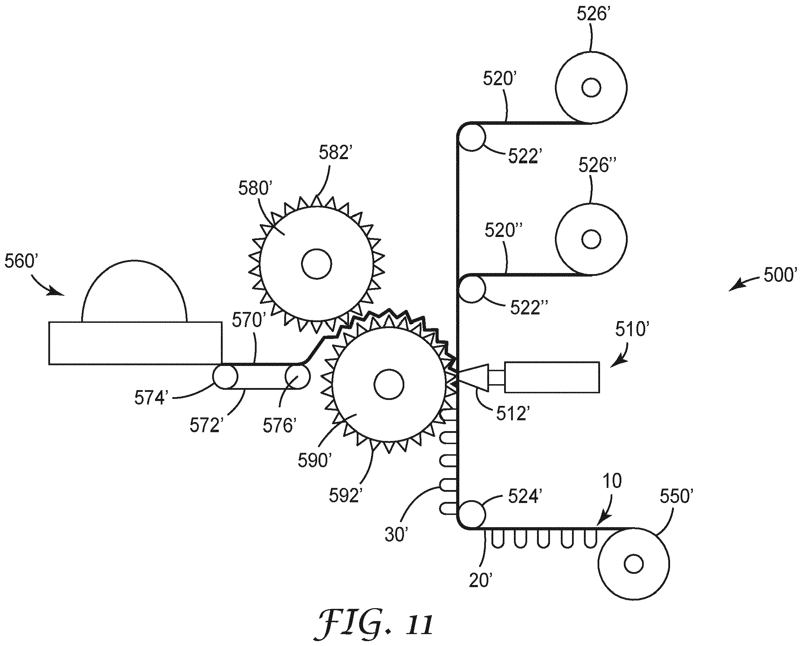

Fig. 11 shows in a schematic functional view the method of making a precursor as shown in

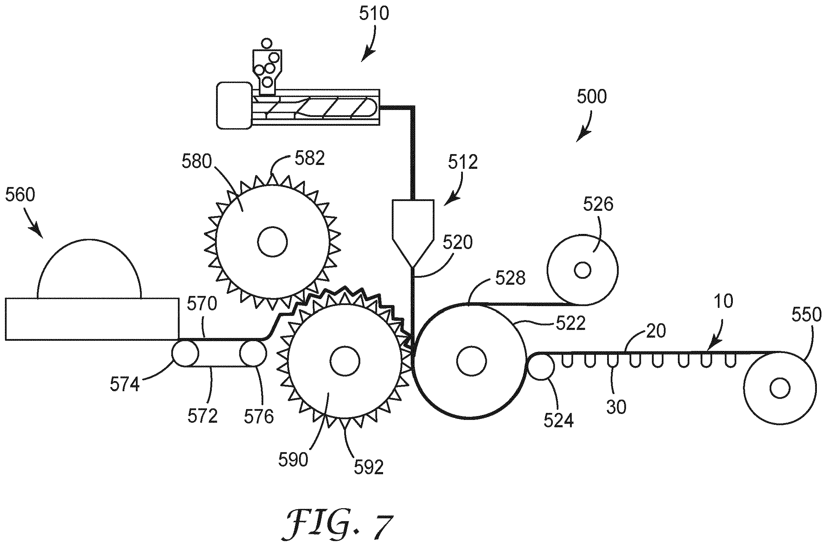

Fig. 7 with a different step of providing a backing and of bonding the fiber web to the backing;

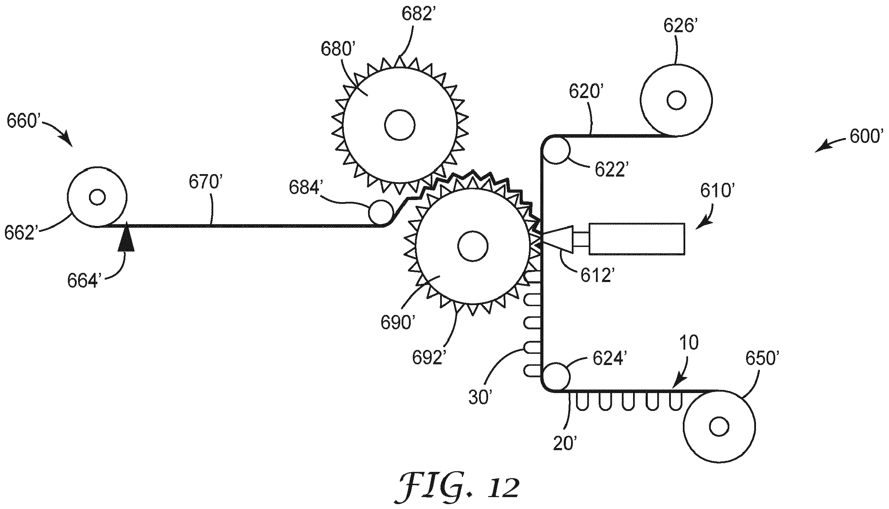

Fig. 12 shows in a schematic functional view the method of making a precursor as shown in

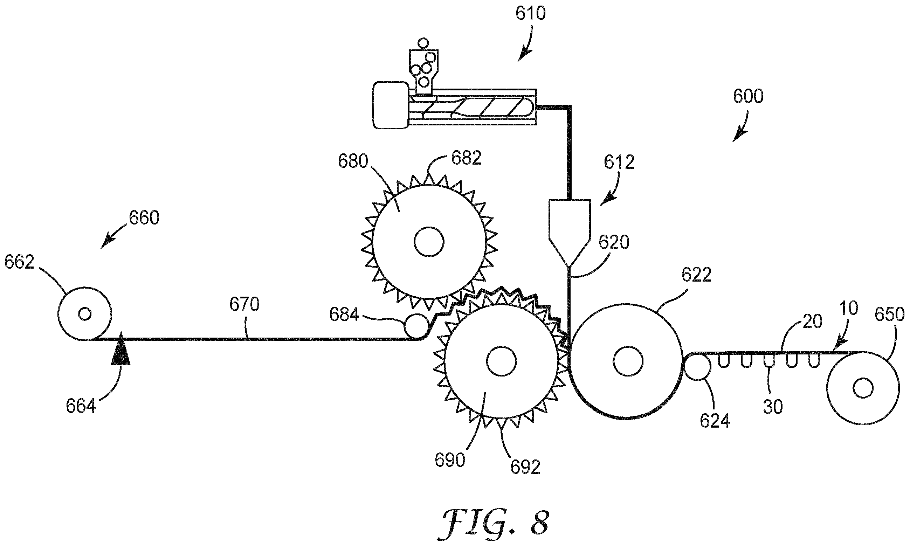

Fig. 8 with a different step of providing a backing and of bonding the fiber web to the backing;

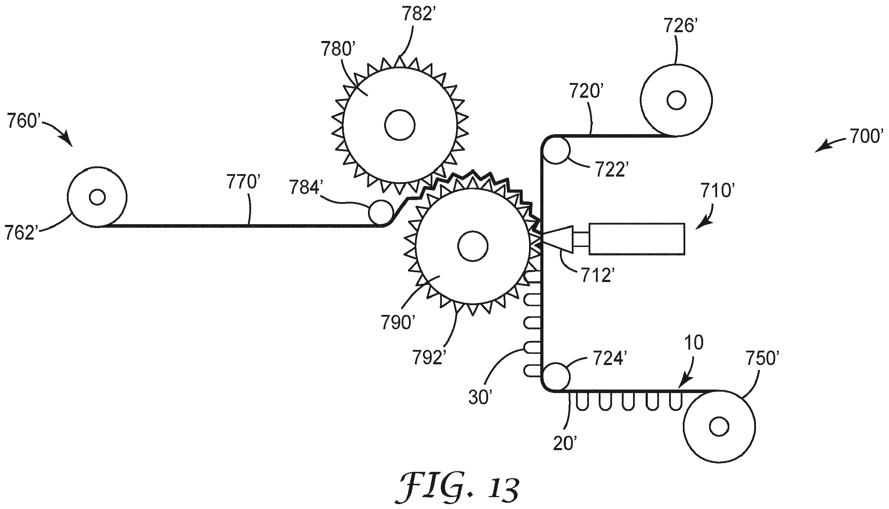

Fig. 13 shows in a schematic functional view the method of making a precursor as shown in

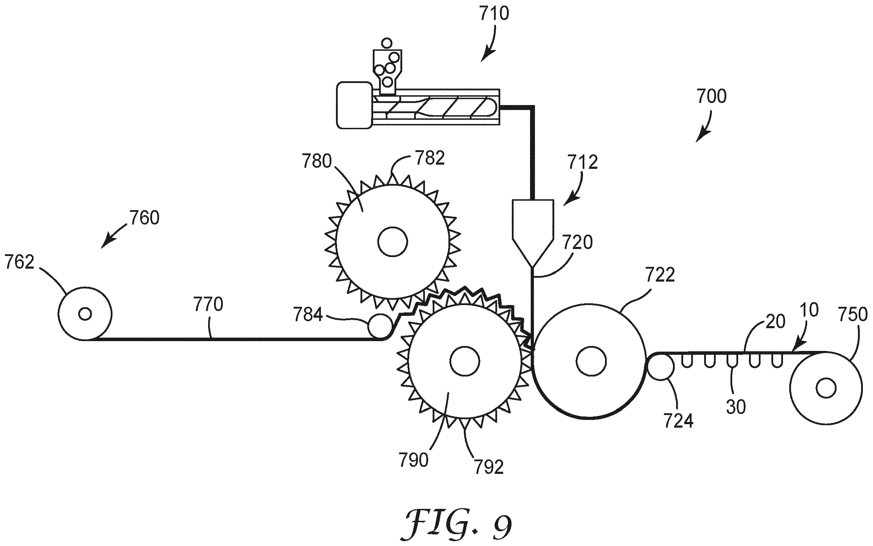

Fig. 9 with a different step of providing a backing and of bonding the fiber web to the backing;

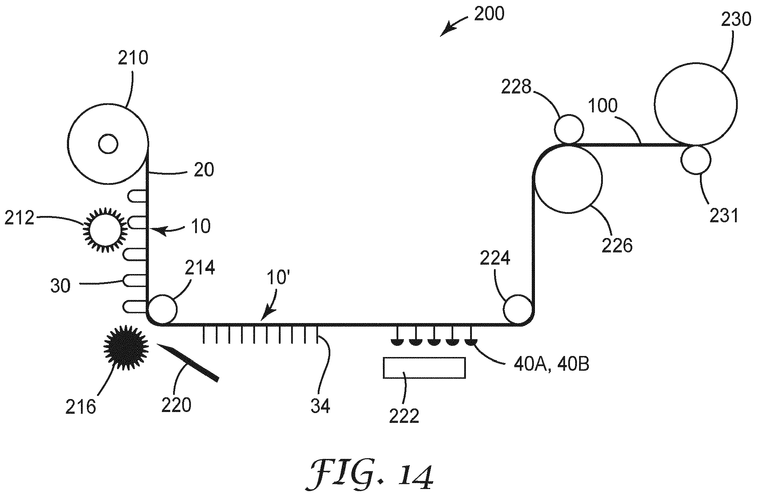

Fig. 14 shows in a schematic functional view a process of providing enlarged portions at the distal ends of the fastening elements according to an embodiment of the present disclosure;

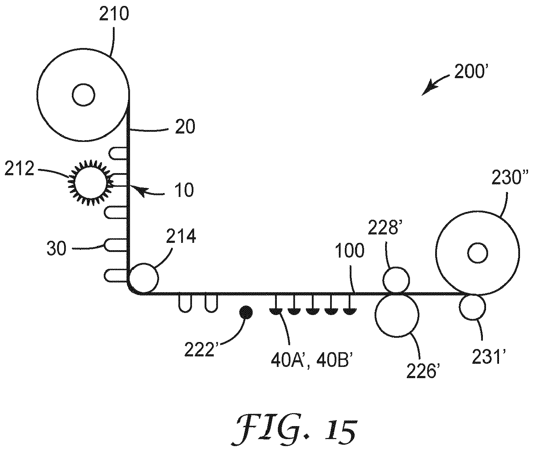

Fig. 15 shows in a schematic functional view a further process of providing enlarged portions at the distal ends of the fastening elements according to an embodiment of the present disclosure;

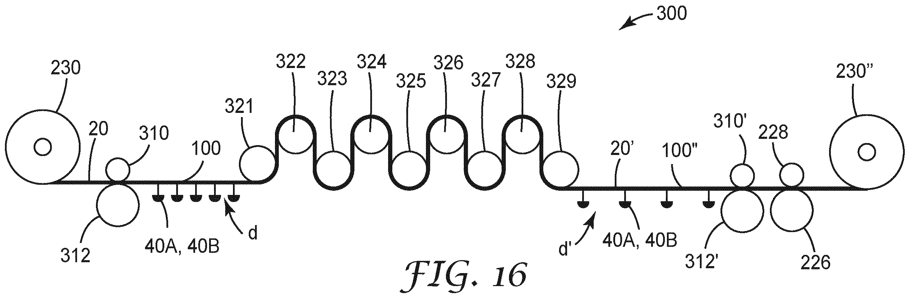

Fig. 16 shows in a schematic functional view a process of separating the fastening elements from each other according to an embodiment of the present disclosure;

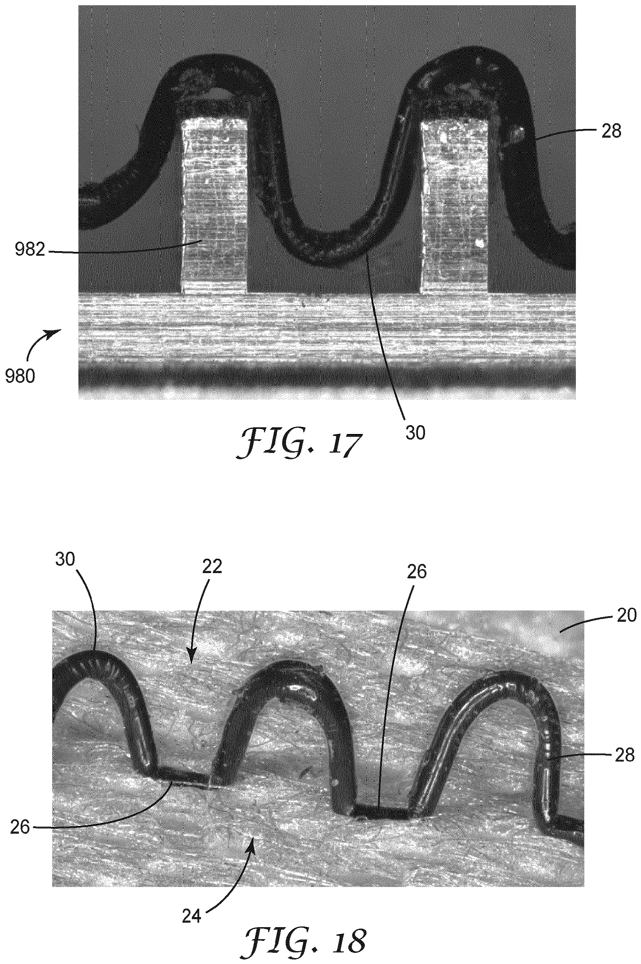

Fig. 17 shows in a microscopy view a detail of a fiber formed in arcuate portions according to the present disclosure prior to bonding to a backing layer;
Fig. 18 shows in a microscopy view a detail of the precursor of the reclosable fastener web according to an embodiment of the present disclosure as shown in Fig. 17 after bonding to a backing layer;

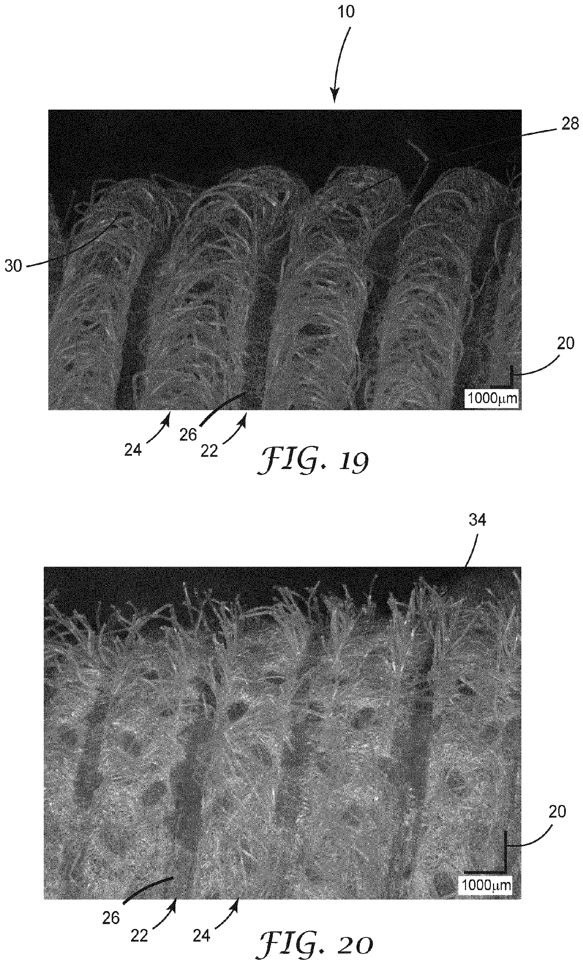

Fig. 19 shows in a microscopy view the precursor of the reclosable fastener web according to an embodiment as shown in Fig. 18, but with a plurality of fibers;
Fig. 20 shows in a microscopy view the precursor of the reclosable fastener web according to an embodiment as shown in Fig. 19 in a later stage;

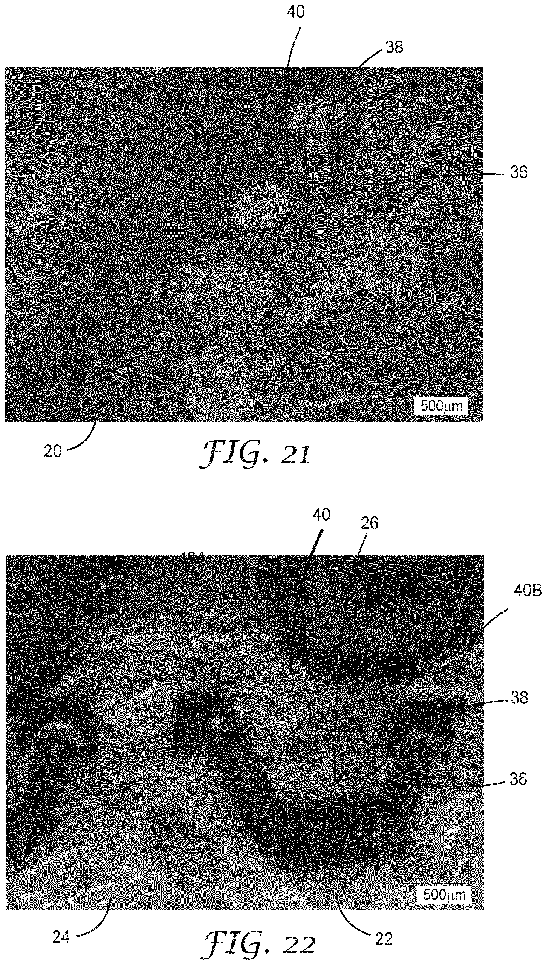

Fig. 21 shows in a microscopy view the U-shaped fasteners of the reclosable fastener web according to an embodiment of the present disclosure;
Fig. 22 shows in a microscopy view the U-shaped fasteners of the reclosable fastener web according to an embodiment of the present disclosure;

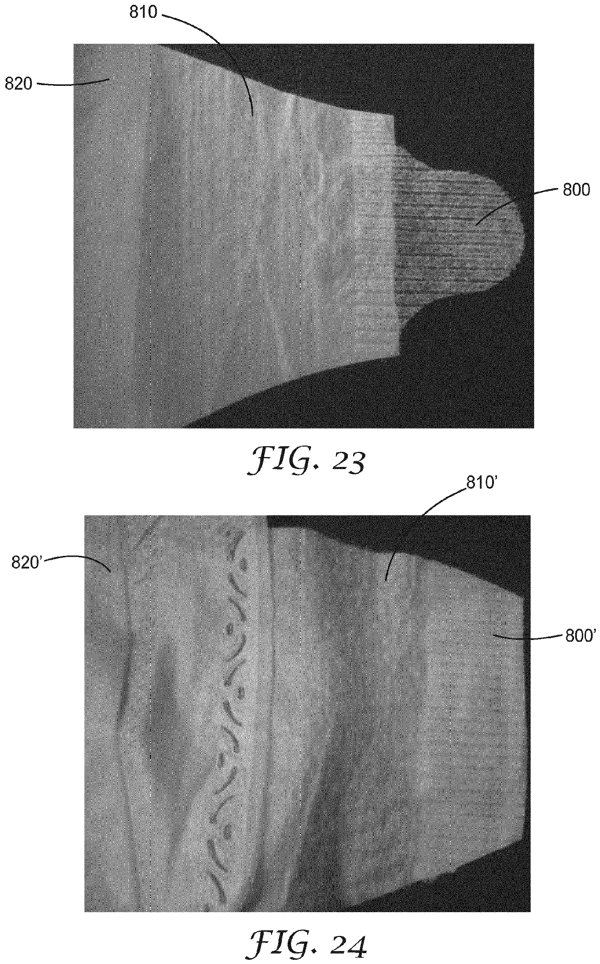

Fig. 23 shows in a top view a part of a hygiene article with an elastic ear with the reclosable fastener web thereon according to an embodiment of the present disclosure;
Fig. 24 shows in a top view a part of a hygiene article with the reclosable fastener web thereon according to an embodiment of the present disclosure;

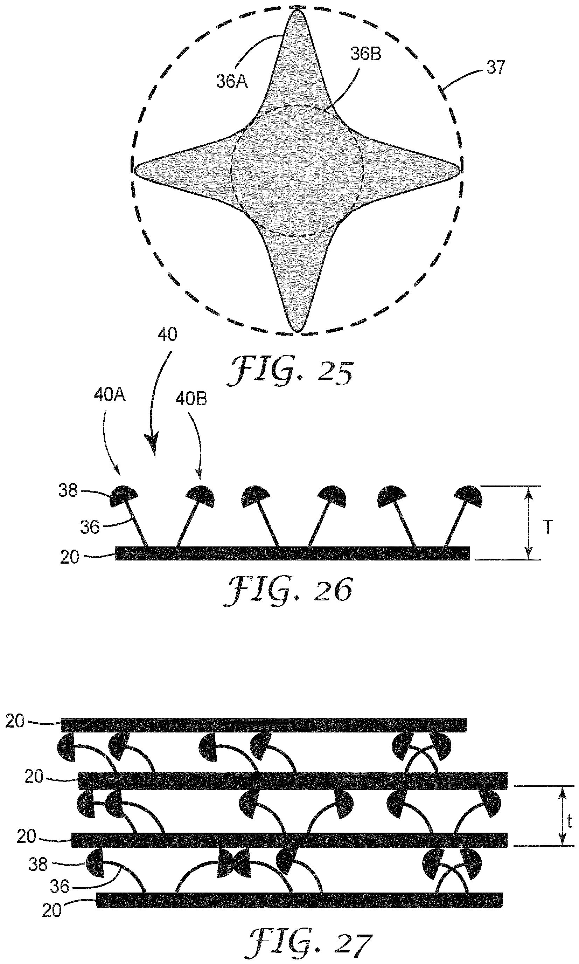

Fig. 25 shows in a schematic cross-sectional view the stem of a fastener of the reclosable fastener web according to an embodiment of the present disclosure;
Fig. 26 shows in a schematic side view a layer of the reclosable fastener web according to an embodiment of the present disclosure before compression;
Fig. 27 shows in a schematic side view several layers of the reclosable fastener web according to an embodiment of the present disclosure after compression.

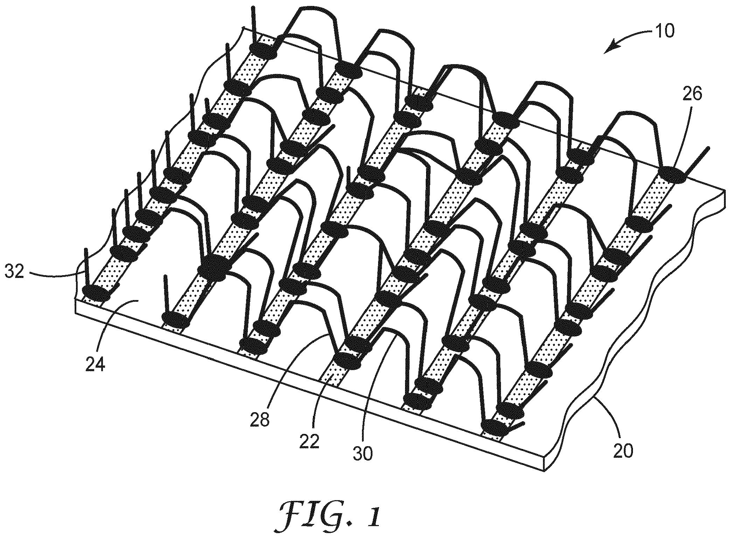

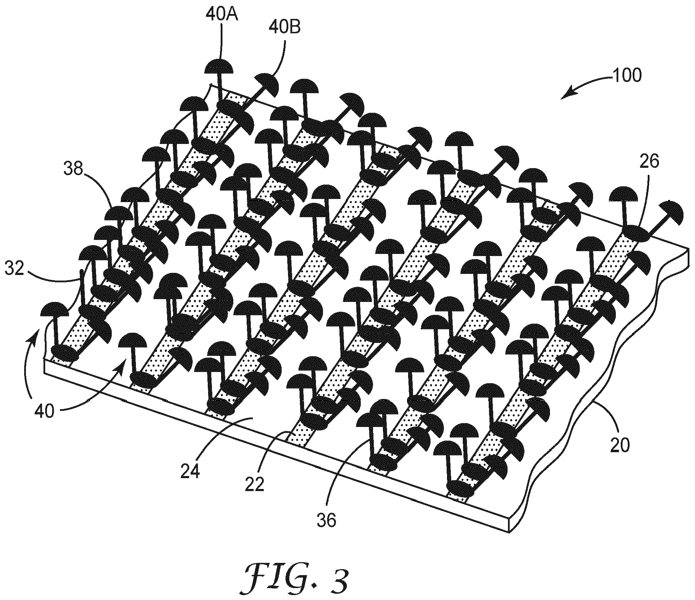

Fig. 1 shows in a schematic perspective view a precursor web 10 of the reclosable fastener web 100 (not shown here, please see Fig. 3). The precursor web 10 comprises a backing layer 20 and fibers bonded thereto formed by the stem portions 28 and the arcuate portions 30. The fibers represent precursors of the U-shaped fasteners 40 (not shown here, please see Fig. 3). The fibers are bonded to the backing layer 20 by elongated bonding areas 22 arranged repeatedly along the extension of the precursor web in a direction perpendicular to the extension of the elongated bonding areas 22. The fibers further comprise flat intermediate portions 26 which connect and attach the fibers to the backing layer 20 at the bonding areas 22. In this stage, as can be seen, the precursor web 10 is substantially formed by continuous fibers extending along the precursor web 10 in a direction perpendicular to the extension of the bonding areas 22 bonded to the backing 20. Some of the fibers may comprise open ends, i. e. not having the arcuate portions 30 present, thereby forming exposed stems 32. The stems 32 may particularly be present at an edge region of the precursor web 10 or may result from a fiber end e. g. in a staple fiber web. Between the elongated bonding areas 22, intermediate spaces 24 are present where substantially no fibers are present and bonded to the backing layer 20, respectively. In the embodiment shown in Fig. 1, the bonding areas 22 and the intermediate spaces 24 are arranged in parallel and intermittently along the extension of the backing layer 20, so that a stripe pattern of bonding areas 22 and intermediate spaces 24 is formed. Other configurations, e. g. where the bonding areas 22 and/or intermediate spaces 24 are not parallelly arranged, are conceivable.

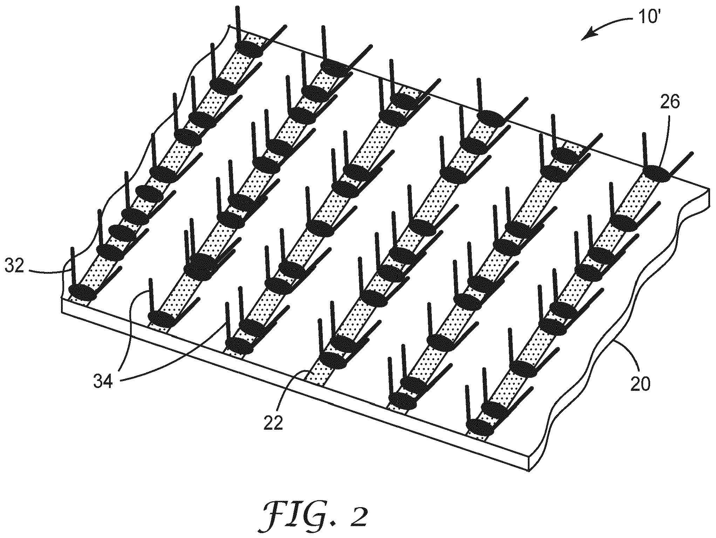

Fig. 2 shows in a schematic perspective view the precursor 10' in a later stage of the manufacture of the reclosable fastener web 100 (not shown here, please see Fig. 3). Here, different to the precursor 10 as shown in Fig. 1, the arcuate portions 30 of the fibers have been cut such that upstanding stems 34 are formed of the meanwhile interrupted fibers bonded to the backing layer 20. Similar to Fig. 1, the precursor 10' of Fig. 2 shows the bonding areas 22 and the intermediate spaces 24 being arranged in parallel and intermittently. Also, stems 32 may be present, particularly in the edge region of the precursor 10. The difference between the stems 32 and stems 34 is that stems 34 have been obtained by cutting the arcuate portions 30 of the fibers, whereas the stems 32 have been in that shape already initially. Fibers with open fiber ends 32 may be cut to the same stem height as a stem resulting from a loop or they remain with a shorter stem height.

Fig. 3 shows in a schematic perspective view the reclosable fastener web 100 which was obtained from the precursor webs 10, 10' as shown in Figs. 1 and 2. The reclosable fastener web 100 comprises U-shaped fasteners 40 which have two fastening elements 40A, 40B both connected to and extending from the flat intermediate portion 26. As mentioned for the precursors or fibers previously for Figs. 1 and 2, the flat intermediate portion 26 connects and attaches the U-shaped fasteners 40 to the backing layer 20 at bonding areas 22. Similar to Figs. 1 and 2, the precursor web10' of Fig. 2 shows the bonding areas 22 and the intermediate spaces 24 being arranged in parallel and intermittently. Each of the U-shaped fasteners 40 comprise two stems 36 and an enlarged portion 38 at the distal ends of the stems 36, which has, for example, been formed by heat treatment of the distal ends of the stems 34 as shown in Fig. 2. Forming the enlarged portions 38 will be explained in more detail further below. While most of the fastening elements are U-shaped and have an enlarged portion 38 on both ends of the stems 36, stems 32 and/or stems 34 may remain that have no enlarged portion. Also, fastening elements 40 may exist that have an enlarged portion on only one of the fastening elements 40A, 40B.

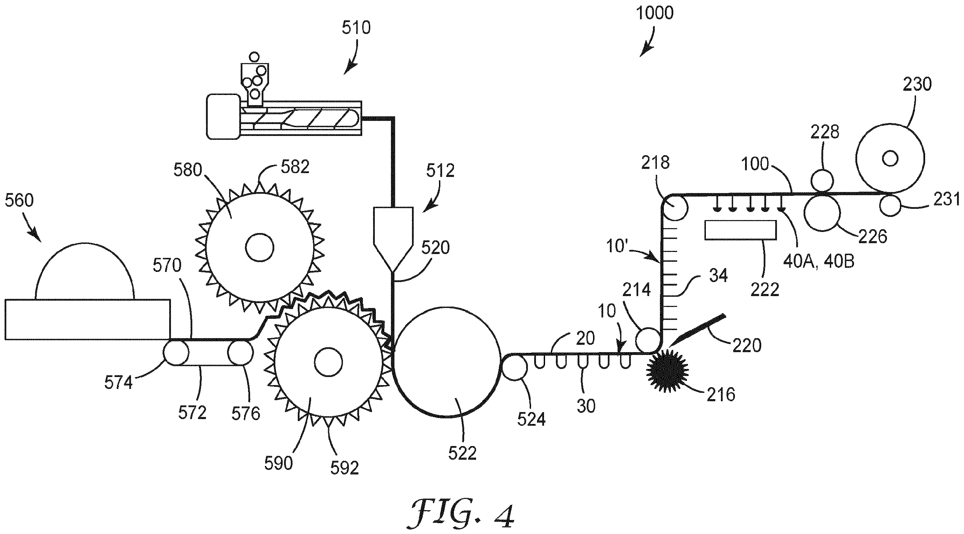

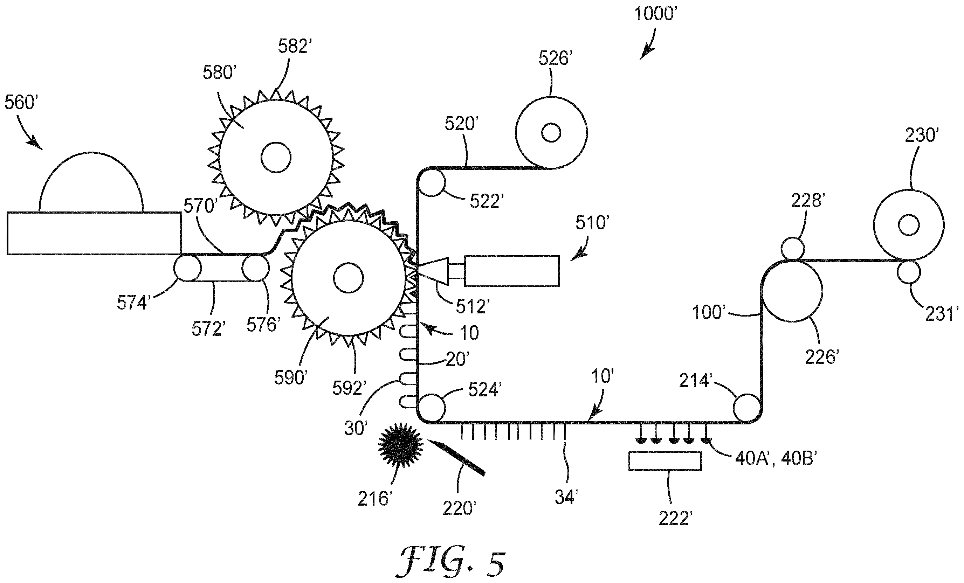

Fig. 4 shows in a schematic functional view an apparatus 1000 for carrying out the method of making a reclosable fastener web 100 as shown in Fig. 3. The method as shown in Fig. 4 represents one option for making the reclosable fastener web 100. A further option for the method will be shown and described in Fig. 5 below. Fig. 4 shows an apparatus 560 for providing a fiber web 570 which is being conveyed on a conveyor belt 572 guided by rollers 574, 576 for further processing. The fiber web 570 will be exposed to a corrugation process by feeding it into a nip between corrugation rollers 580, 590 having a corrugated surface 582, 592. After corrugating the fiber web 570, a backing layer 20 is formed by extrusion with an extruder 510 having an extrusion nozzle 512 for extruding a molten film, i. e. the backing layer material 520 later forming the backing layer 20. It is noted that the backing layer material 520 in this stage is molten, whereas the backing layer 20 in a later stage is substantially solidified. The backing layer material 520 is then brought into contact with the corrugated fiber web 570 on the corrugation roller 590 and its corrugated surface 592, respectively, in such a way that the tip portions of the corrugated fiber web 570 are being partially embedded into the still molten backing layer material 520. Bonding regions 22 (not shown here) as shown in Figs. 1 to 3, are formed thereby, at which the fiber web 570 is bonded to the backing layer material 520 and the later formed and solidified backing layer 20. Guide roller 522 provides the required pressure between the backing layer material 520 and the corrugated fiber web 570. Cooling down of the molten backing layer material 520 is taking place, wherein the guide roller 522 may at the same time apply an additional cooling onto the backing layer material 520 to enhance the solidification thereof. The so obtained composite of the corrugated fiber web 570 and the backing layer material 520 bonded to each other results - after cooling down - in backing layer 20 with arcuate portions 30 of the fiber web 570 thereon. Guide roller 524 helps to facilitate a reliable web transport for further processing. The arcuate portions 30 of the fiber web 570 bonded to the backing layer 20 are then cut between a rotating cutter 216 and a ledger blade 220, wherein guiding roller 214 facilitates reliable web transport here thereby forming the precursor web 10'. The so obtained precursor web 10' is then being further transported by guide roller 218 and being subjected to a heat treatment by heater 222 applying heat to the distal ends of the stems 34 to form fastening elements 40A, 40B. It is noted that the fastening elements 40A, 40B form the U-shaped fasteners 40, which is not visible here, but which is shown in Fig. 3 above. The so obtained reclosable fastener web 100 is then processed through a length cutting station comprising a cutting roller 228 and a counter roller 226 and then wound up by to a storage or supply roll 230 of reclosable fastener web 100, wherein roller 231 facilitates reliable web transport and compression of the fastener web.

Fig. 5 shows in a schematic functional view an apparatus 1000' for carrying out the method of making a reclosable fastener web 100' as shown in Fig. 3. The method as shown in Fig. 5 represents a further option for making the reclosable fastener web 100 and differs from the method as illustrated in Fig. 4. The main difference is the provision of the backing layer 520', which is unwound from a supply roll 526' and guided over roller 522' to be fed to the bonding process. Also, the bonding process is different to Fig. 4, i. e. the differently provided backing layer 520', which is not in a molten state as backing layer 520 in Fig. 4, but solidified when fed to the corrugated fiber web 570'. The bonding in this case is made by an ultrasonic bonding device 510' comprising an ultrasonic horn 512', through which ultrasonic energy is applied on the backing layer 520' with the laid over corrugated fiber web 570' such that the heat resulting from the applied ultrasonic energy bonds the fiber web 570', i. e. the tips of the corrugations as created by the rollers 580', 590' with their corrugated surfaces 582', 592'. Fig. 5 shows an apparatus 560' for providing a fiber web 570' which is being conveyed on a conveyor belt 572' guided by roller 574', 576' for further processing. The fiber web 570' will be exposed to a corrugation process by feeding it into a nip between corrugation rollers 580', 590' each having a corrugated surface 582', 592'. After corrugating the fiber web 570' as mentioned above, a backing layer 20' is provided by unwinding the backing layer material 520' from the supply roll 526' and guiding it hereto over guide roller 522'. It is noted that the backing layer material 520' in this stage will be bonded through the application of ultrasonic energy as described above to the corrugated fiber web 570' wherein the provided backing layer material 520' represents the backing layer 20'. Bonding regions 22 (not shown here) as shown in Figs. 1 to 3, are formed thereby, at which the fiber web 570 is bonded to the backing layer material 520' and the backing layer 20', respectively. Different to Fig. 4, no guide roller 522 for providing the required pressure between the backing layer material 520' and the corrugated fiber web 570' and no cooling down of the backing layer material is taking place or needed here. Guide roller 524' helps to facilitate a reliable web transport for further processing. The precursor web 10 is formed thereby. The arcuate portions 30' of the fiber web 570' bonded to the backing layer 20' are then cut between a rotating cutter 216 and a ledger blade 220, wherein guiding roller 524' facilitates reliable web transport here. The precursor web 10' is formed thereby. The so obtained precursor web 10' is then being further transported and being subjected to a heat treatment by heater 222' applying heat to the distal ends of the stems 34' to form fastening elements 40A', 40B', wherein guiding roller 214' facilitates reliable web transport here. It is noted that the fastening elements 40A', 40B' form the U-shaped fasteners 40', which is not visible here, but which is shown in Fig. 3 above. The so obtained reclosable fastener web 100' is then processed through a length cutting station comprising a cutting roller 228' and a counter roller 226' and then wound up into a storage or supply roll 230' of reclosable fastener web 100', wherein roller 231' facilitates reliable web transport and compression of the reclosable fastener web 100'.

Fig. 6 shows in a schematic functional view a method of making a precursor 10 of the reclosable fastener web 100 (not visible here) according to an embodiment of the present disclosure. Fig. 6 shows one option for the method, whereas further methods are shown and described below in Figs. 7 to 9. The apparatus 400 comprises a fiber providing apparatus 460 which provides a plurality of unwind stations for spools 462A-C of fiber (e. g. monofilament or multifilament). Fibers from several spools can be bundled and guided by a guiding roller 463 to a comb 464. The comb 464 covers the entire width of the fiber web and defines the spacing of the fibers in the fiber web. In Fig. 6 three guiding rollers are shown, each guiding fibers from three spools. It is understood that for a wide production width a much higher number of fibers is required. The fiber web is guided by roller 484 into the corrugation nip between the corrugation rollers 480, 490 each having a corrugated surface 482, 492. After corrugating the fiber web 470, a backing layer 20 is formed by extrusion with an extruder 410 having an extrusion nozzle 412 for extruding a molten film, i. e. the backing layer material 420 later forming the backing layer 20. It is noted that the backing layer material 420 in this stage is molten, whereas the backing layer 20 in a later stage is substantially solidified. The backing layer material 420 is then brought into contact with the corrugated fiber web 470 on the corrugation roller 490 and its corrugated surface 492, respectively, in such a way that the tip portions of the corrugated fiber web 470 are being partially embedded into the still molten backing layer material 420. Bonding regions 22 (not shown here) as shown in Figs. 1 to 3, are formed thereby, at which the fiber web 470 is bonded to the backing layer material 420 and the later formed and solidified backing layer 20. Guide roller 422 provides the required pressure between the backing layer material 420 and the corrugated fiber web 470 thereby forming the flat intermediate portions 26 (not visible here, please see Figs. 1 to 3). Cooling down of the molten backing layer material 420 is taking place wherein the guide roller 422 may at the same time apply an additional cooling to the backing layer material 420. The so obtained composite of the corrugated fiber web 470 and the backing layer material 420 bonded to each other results - after cooling down - in backing layer 20 with arcuate portions 30 of the fiber web 470 thereon. Guide roller 424 helps to facilitate a reliable web transport for further processing, in the example shown in Fig. 6 this is the winding up of the obtained precursor web 10 onto storage roll 450.

Fig. 7 shows in a schematic functional view a further method of making a precursor 10 of the reclosable fastener web 100 (not visible here) according to an embodiment of the present disclosure. Fig. 7 shows a further option for the method, whereas further methods are shown and described in Fig. 6 above as well as in Figs. 8 and 9 below. The apparatus 500 comprises an apparatus 560 for providing a fiber web 570 which is being conveyed on a conveyor belt 572 guided by roller 574, 576 for further processing. The fiber web 570 will be exposed to a corrugation process and is therefore fed into a nip of corrugation rollers 580, 590 each having corrugated surfaces 582, 592. After corrugating the fiber web 570, a backing layer 20 is formed by extrusion with an extruder 510 having an extrusion nozzle 512 for extruding a molten film, i. e. the backing layer material 520 later forming the backing layer 20. It is noted that the backing layer material 520 in this stage is molten, whereas the backing layer 20 in a later stage is substantially solidified. The backing layer material 520 is then brought into contact with the corrugated fiber web 570 on the corrugation roller 590 and its corrugated surface 592, respectively, in such a way that the tip portions of the corrugated fiber web 570 are being partially embedded into the still molten backing layer material 520. Bonding regions 22 (not shown here) as shown in Figs. 1 to 3 are formed thereby, at which the fiber web 570 is bonded to the backing layer material 520 and the later formed and solidified backing layer 20. Guide roller 522 provides the required pressure between the backing layer material 520 and the corrugated fiber web 570 such that the flat intermediate portions 26 (not visible here, please see Figs. 1 to 3) are formed thereby. Cooling down of the molten backing layer material is taking place, wherein the guide roller 522 may at the same time apply an additional cooling onto the backing layer material 520. Different to the process as shown in Fig. 4, an additional backing layer 528 is being unwound from the supply roll 526 and then being fed into the nip between corrugation roller 590 with its corrugated surface 592 and the guide roller 522 and thereby being brought into contact with the molten backing layer material 520 opposite to the side where the corrugated fiber web 570 is contacting the backing layer material 520. The backing layer material 520 is thus being sandwiched between the corrugated fiber web 570 and the additional backing layer 528. The so obtained composite of the corrugated fiber web 570 and the backing layer material 520 bonded to each other results - after cooling down - in backing layer 20 with arcuate portions 30 of the fiber web 570 thereon. Because the backing layer material 520 is still in a molten state, bonding between the backing layer 528 and the backing layer material 520 is achieved at the same time by cooling down of the backing layer material, similarly to the bonding and cooling down of the backing layer material 520 and the corrugated fiber web 570 as described here. As described above, the guide roller 522 may apply an additional cooling onto the backing layer material 520 with the additional backing layer 528 being in contact therewith. Guide roller 524 helps to facilitate a reliable transport of the so obtained precursor web 10 for further processing, in the example shown in Fig. 6 this is the winding up of the obtained precursor web 10 onto storage roll 550.

Fig. 8 shows in a schematic functional view another method of making a precursor 10 of the reclosable fastener web 100 (not visible here) according to an embodiment of the present disclosure. Fig. 8 shows a further option for the method, whereas further methods are shown and described in Figs 6 and 7 above as well as in Fig. 9 below. The apparatus 600 comprises a fiber web apparatus 660, in which a fiber web 670 is being unwound from the supply roll 662 and is being subjected to a comb 664 for filament guiding. The so treated fiber web 670 is then being guided with roller 684 for further web processing. The further web processing includes that the fiber web 670 is fed into a nip of corrugation rollers 680, 690 each having corrugated surface 682, 692. After corrugating the fiber web 670, a backing layer 20 is formed by extrusion with an extruder 610 having an extrusion nozzle 612 for extruding a molten film, i. e. the backing layer material 620 later forming the backing layer 20.

It is noted that the backing layer material 620 in this stage is molten, whereas the backing layer 20 in a later stage is substantially solidified. The backing layer material 620 is then brought into contact with the corrugated fiber web 670 on the corrugation roller 690 and its corrugated surface 692, respectively, in such a way that the tip portions of the corrugated fiber web 670 are being partially embedded into the still molten backing layer material 620. Bonding regions 22 (not shown here) as shown in Figs. 1 to 3 are formed thereby, at which the fiber web 670 is bonded to the backing layer material 620 and the later formed and solidified backing layer 20. Guide roller 622 provides the required pressure between the backing layer material 620 and the corrugated fiber web 670 so that the flat intermediate portions 26 (not visbible here, please see Figs. 1 to 3) are formed. Cooling down of the molten backing layer material is taking place, wherein the guide roller 622 may at the same time apply an additional cooling onto the backing layer material 620. The so obtained composite of the corrugated fiber web 670 and the backing layer material 620 bonded to each other results - after cooling down - in backing layer 20 with arcuate portions 30 of the fiber web 670 thereon. Guide roller 624 helps to facilitate a reliable transport of the so obtained precursor web 10 for further processing, in the example shown in Fig. 6 this is the winding up of the obtained precursor web 10 onto storage roll 650.

Fig. 9 shows in a schematic functional view still another method of making a precursor 10 of the reclosable fastener web 100 (not visible here) according to an embodiment of the present disclosure. Fig. 9 shows a further option for the method, whereas further methods are shown and described above in Figs. 6 to 8. The apparatus 700 comprises a web provision apparatus 760 having a supply roll 762 from which a fiber web, in particular a nonwoven web 770 is being unwound. The nonwoven web 770 is being further guided by the guide roll 784 for further web processing. The further web processing includes that the nonwoven web 770 is fed into a nip of corrugation rollers 780, 790 each having corrugated surfaces 782, 792. After corrugating the nonwoven web 770, a backing layer 20 is formed by extrusion with an extruder 710 having an extrusion nozzle 712 for extruding a molten film, i. e. the backing layer material 720 later forming the backing layer 20. It is noted that the backing layer material 720 in this stage is molten, whereas the backing layer 20 in a later stage is substantially solidified. The backing layer material 720 is then brought into contact with the corrugated nonwoven web 770 on the corrugation roller 790 and its corrugated surface 792, respectively, in such a way that the tip portions of the corrugated nonwoven web 770 are being partially embedded into the still molten backing layer material 720. Bonding regions 22 (not shown here) as shown in Figs. 1 to 3, are formed thereby, at which the nonwoven web 770 is bonded to the backing layer material 720 and the later formed and solidified backing layer 20. Guide roller 722 provides the required pressure between the backing layer material 720 and the corrugated nonwoven web 770 such that the flat intermediate portions 26 (not visible here, please see Figs. 1 to 3) are formed. Cooling down of the molten backing layer material is taking place, wherein the guide roller 722 may at the same time apply an additional cooling onto the backing layer material 720. The so obtained composite of the corrugated nonwoven web 770 and the backing layer material 720 bonded to each other results - after cooling down - in backing layer 20 with arcuate portions 30 of the nonwoven web 770 thereon. Guide roller 724 helps to facilitate a reliable transport of the so obtained precursor web 10 for further processing, in the example shown in Fig. 6 is that the winding up of the precursor web 10 onto storage roll 750.

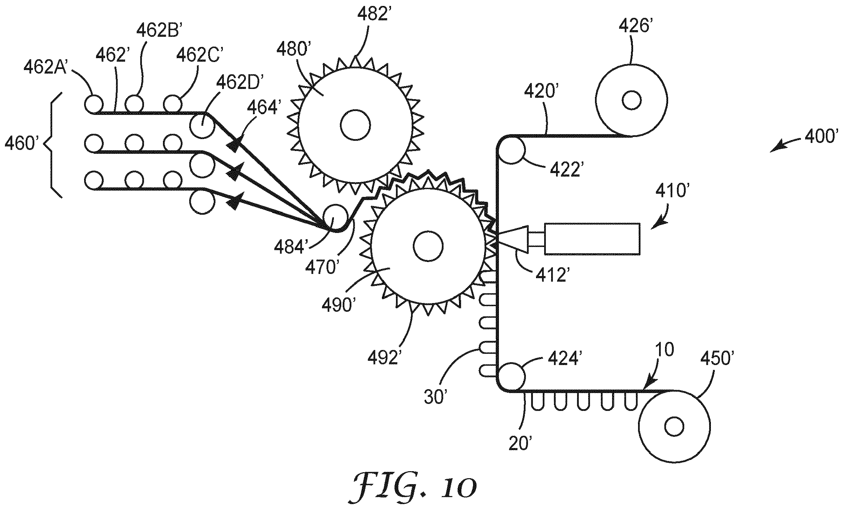

Fig. 10 shows in a schematic functional view the method of making a precursor 10 as shown in Fig. 6 with a different step of providing a backing 420' and of bonding the fiber web 470' to the backing 420'. Fig. 10 shows one option for the method, whereas further methods are shown and described below in Figs. 11 to 13. The apparatus 400' comprises a fiber providing apparatus 460' which provides a plurality of unwind stations for spools 462A-C of fiber (e. g. monofilament or multifilament). Fibers from several spools can be bundled and guided by a guiding roller 463' to a comb 464'. The comb 464' covers the entire width of the fiber web and defines the spacing of the fibers in the fiber web. In Fig. 10 three guiding rollers are shown, each guiding fibers from three spools. It is understood that for a wide production width a much higher number of fibers is required. The fiber web is guided by roller 484 into the corrugation nip of corrugation rollers 480', 490' each having corrugated surface 482', 492'. The main difference to the process as shown in Fig. 6 is the provision of the backing layer 420', which is unwound from a supply roll 426' and guided over roller 422' to be fed to the bonding process. Also, the bonding process is different to Fig. 6, i. e. the differently provided backing layer 420', which is not in a molten state as backing layer 420 in Fig. 6 but is solidified when fed to the corrugated fiber web 470'. The bonding in this case is made by an ultrasonic bonding device 410' comprising an ultrasonic horn 412', through which ultrasonic energy is applied on the backing layer 420' with the laid over corrugated fiber web 470' such that the heat resulting from the applied ultrasonic energy bonds the fiber web 470', i. e. the tips of the corrugations as created by the rollers 480', 490' with their corrugated surfaces 482', 492'. Bonding regions 22' (not shown here) as shown in Figs. 1 to 3, are formed thereby, at which the fiber web 470' is bonded to the backing layer 420' thereby forming the backing layer 20' with the arcuate portions 30' of the fiber web 470' and thereby forming the flat intermediate portions 26 (not visible here, please see Figs. 1 to 3). Guide roller 424' helps to facilitate a reliable transport of the precursor web 10 for further processing, in the example shown in Fig. 10 this is the winding up of the obtained precursor web 10 onto storage roll 450'.

Fig. 11 shows in a schematic functional view the method of making a precursor 10 as shown in Fig. 7 with a different step of providing a backing 520' and of bonding the fiber web 570' to the backing 520'. Similar to Fig. 7, an additional backing layer 520" is being unwound from the supply roll 526". The additional backing layer 520" is then being guided by the guide roller 522" and being fed into the nip between corrugation roller 590' with its corrugated surface 592' and the guide roller 522' and thereby being brought into contact with the backing layer 520' opposite to the side where the corrugated fiber web 570' is contacting the backing layer 520'. The backing layer 520' is thus being sandwiched between the corrugated fiber web 570' and the additional backing layer 520". The so obtained composite of the corrugated fiber web 570' and the backing layers 520', 520" bonded to each other form the backing layer 20' with arcuate portions 30' of the fiber web 570' thereon. Fig. 11 shows one option for the method, whereas further methods are shown and described in Figs. 10, 12 and 13 below. The apparatus 500' comprises an apparatus 560' for providing a fiber web 570' which is being conveyed on a conveyor 572' guided by roller 574', 576' for further processing. The fiber web 570' will be exposed to a corrugation process and is therefore being fed into a nip of corrugation rollers 580', 590' each having corrugated surfaces 582', 592'. The main difference to the process as shown in Fig. 7 is the provision of the backing layer 520', which is unwound from a supply roll 526' and guided over roller 522' to be fed to the bonding process. Also, the bonding process is different to Fig. 7, i. e. the differently provided backing layer 520', which is not in a molten state as backing layer 520 in Fig. 7 but is solidified when fed to the corrugated fiber web 570'. As mentioned above, another difference to the process of Fig. 7 is that an additional backing layer 520" is unwound from a supply roll 526" and fed to the composite as formed by the corrugated fiber web 570' and the backing layer 520' and is bonded thereto at a side opposite to where the corrugated fiber web 570' is being bonded to by the ultrasonic bonding process described below. The bonding in this case is made by an ultrasonic bonding device 510' comprising an ultrasonic horn 512', through which ultrasonic energy is applied on the backing layers 520', 520" with the laid over corrugated fiber web 570' such that the heat resulting from the applied ultrasonic energy bonds the fiber web 570', i. e. the arcuate portions 30' of the corrugations as created by the rollers 580', 590' with their corrugated surfaces 582', 592'. Bonding regions 22' (not shown here) as shown in Figs. 1 to 3 are formed thereby, at which the fiber web 570' is bonded to the backing layers 520', 520" thereby forming the backing layer 20' with the arcuate portions 30' of the fiber web 570' and thereby forming the flat intermediate portions 26 (not visible here, please see Figs. 1 to 3). Guide roller 524' helps to facilitate a reliable transport of the so obtained precursor web 10 for further processing, in the example shown in Fig. 11 this is the winding up of the obtained precursor web 10 onto storage roll 550'.

Fig. 12 shows in a schematic functional view the method of making a precursor 10 as shown in Fig. 8 with a different step of providing a backing 620' and of bonding the filaments 670' to the backing 620'. Fig. 12 shows one option for the method, whereas further methods are shown and described in Figs. 10, 11 and 13 below. The apparatus 600' comprises a fiber web apparatus 660', in which a fiber web 670' is being unwound from the supply roll 662' and is being subjected to a comb 664' for filament guiding. The so treated fiber web 670' is then being guided with roller 684' for further web processing such that the fiber web 670' is fed into a nip of corrugation rollers 680', 690' each having corrugated surface 682', 692'. The main difference to the process as shown in Fig. 8 is the provision of the backing layer 620', which is unwound from a supply roll 626' and guided over roller 622' to be fed to the bonding process. Also, the bonding process is different to Fig. 8, i. e. the differently provided backing layer 620', which is not in a molten state as backing layer 620 in Fig. 8, but is solidified when fed to the corrugated fiber web 670'. The bonding in this case is made by an ultrasonic bonding device 610' comprising an ultrasonic horn 612', through which ultrasonic energy is applied on the backing layer 620' with the laid over corrugated fiber web 670' such that the heat resulting from the applied ultrasonic energy bonds the fiber web 670', i. e. the tips of the corrugations as created by the rollers 680', 690' with their corrugated surfaces 682', 692'. Bonding regions 22' (not shown here) as shown in Figs. 1 to 3 are formed thereby, at which the fiber web 670' is bonded to the backing layer 620' thereby forming the backing layer 20' with the arcuate portions 30' of the fiber web 670' and thereby forming the flat intermediate portions 26 (not visible here, please see Figs. 1 to 3). Guide roller 624' helps to facilitate a reliable transport of the so obtained precursor web 10 for further processing, in the example shown in Fig. 10 this is the winding up of the obtained precursor web 10 onto storage roll 650'.

Fig. 13 shows in a schematic functional view the method of making a precursor 10 as shown in Fig. 9 with a different step of providing a backing 720' and of bonding the fiber web, in particular the nonwoven web 770' to the backing 720'. Fig. 13 shows one option for the method, whereas further methods are shown and described in Figs. 10 to 12 above. The apparatus 700' comprises a web provision apparatus 760' having a supply roll 762' from which a fiber web, in particular a nonwoven web 770' is being unwound. The nonwoven web 770' is being further guided by the guide roll 784' for further web processing. The further web processing includes that the nonwoven web 770' is fed into a nip of corrugation rollers 780', 790' each having corrugated surface 782', 792'. The main difference to the process as shown in Fig. 9 is the provision of the backing layer 720', which is unwound from a supply roll 726' and guided over roller 722' to be fed to the bonding process. Also, the bonding process is different to Fig. 9, i. e. the differently provided backing layer 720', which is not in a molten state as backing layer 720 in Fig. 9, but is solidified when fed to the corrugated fiber web 770'. The bonding in this case is made by an ultrasonic bonding device 710' comprising an ultrasonic horn 712', through which ultrasonic energy is applied on the backing layer 720' with the laid over corrugated fiber web 770' such that the heat resulting from the applied ultrasonic energy bonds the fiber web 770', i. e. the tips of the corrugations as created by the rollers 780', 690' with their corrugated surfaces 782', 792'. Bonding regions 22' (not shown here) as shown in Figs. 1 to 3 are formed thereby, at which the fiber web 770' is bonded to the backing layer 720' thereby forming the backing layer 20' with the arcuate portions 30' of the fiber web 770' and thereby forming the flat intermediate portions 26 (not visible here, please see Figs. 1 to 3). Guide roller 724' helps to facilitate a reliable transport of the so obtained precursor web 10 for further processing, in the example shown in Fig. 10 this is the winding up of the obtained precursor web 10 onto storage roll 750'.

Fig. 14 shows in a schematic functional view a process of providing enlarged portions 38 at the distal ends of the fastening elements 40A, 40B of the U-shaped fasteners 40 according to an embodiment of the present disclosure. The process as illustrated in Fig. 14 is part of the method of making a reclosable fastener web 100 as illustrated and described in Figs. 4 and 5. The difference of the process as laid out in Fig. 14 to that as laid out in Figs. 4 and 5 is that the precursor web 10 has been wound up intermediately after making the precursor web 10, i. e. without continuing of the complete process as laid out in Figs. 4 and 5. As can be seen in Fig. 14, the backing 20 of the precursor web 10 of the reclosable fastener web 100 (not visible here) is being unwound from a supply roll 210 onto which the precursor web 10 had previously been wound up for storage after making the precursor web 10. The backing 20 comprises the arcuate portions 30, which is described in more detail in Fig. 1. A brush roller 212 is arranged at which the loops of the backing 20 of the precursor web 10 are raised for further processing. This step may be beneficial as the loops of the precursor web 10 may have been squeezed and compressed or flattened due to winding up and storing the precursor web 10 on the storage roll 210, particularly if stored for an elongated period of time. The precursor web 10 with the raised loops comprising arcuate portions 30 is then guided over a guide roller 214 for cutting. The arcuate portions 30 of the fiber web bonded to the backing layer 20 are then cut between a rotating cutter 216 and a ledger blade 220, wherein guiding roller 214 facilitates reliable web transport here thereby forming the precursor web 10'. The precursor web 10' web is then being subjected to a heat treatment by heater 222 applying heat to the distal ends of the stems 34 to form fastening elements 40A, 40B. It is noted that the fastening elements 40A, 40B form the U-shaped fasteners 40, which is not visible here, but which is shown in Fig. 3 above. The so obtained reclosable fastener web 100 is then processed through a length cutting station comprising a cutting roller 228' and a counter roller 226' and then wound up into a roll 230 of reclosable fastener web 100, wherein roller 231 facilitates reliable web transport and compression of the fastener web 100.

Fig. 15 shows in a schematic functional view a further process being different as compared to Fig. 14 by providing enlarged portions 38 at the distal ends of the fastening elements 40A, 40B of the U-shaped fasteners 40 according to an embodiment of the present disclosure. As mentioned above, the process as shown in Fig. 14 is also part of the method of making a reclosable fastener web 100 as illustrated and described in Figs. 4 and 5. The difference of the process as laid out in Fig. 14 to that as laid out in Figs. 4 and 5 is that the precursor web 10 has been wound up intermediately after making the precursor web 10, i. e. without continuing of the complete process as laid out in Figs. 4 and 5. It is noted that this process may also be part of the entire process as shown in Figs. 4 and 5 instead of the process step for making the enlarged portions 38 as illustrated in Fig. 14. As can be seen in Fig. 15, the backing 20 of the precursor web 10 of the reclosable fastener web 100 is being unwound from a supply roll 210 onto which the precursor web 10 had previously been wound up for storage after making the precursor web 10. The backing 20 comprises the arcuate portions 30, which is described in more detail in Fig. 1. A brush roller 212 is arranged at which the loops of the backing 20 of the precursor web 10 are raised for further processing. This step may be beneficial as the loops of the precursor web 10 may have been squeezed and compressed or flattened due to winding up and storing the precursor web 10 on the storage roll 210, particularly if stored for an elongated period of time. The precursor web 10 with the raised loops formed by the arcuate portions 30 and the stem portions 28 is then guided over a guide roller 214 for further processing. Different to the process as shown in Fig. 14, where the arcuate portions 30 of the fiber bonded to the backing layer 20 are being cut by a cutter system 220 and 216 to increase the number of fiber ends and to provide stems 34, the web is then directly being subjected to a heat treatment by a hot wire 222'. The heat of the hot wire 222' cuts the arcuate portions of the fibers and thereby directly forms the enlarged portions 38 at the distal end of the resulting stems 40A' and 40B', i. e. no separate cutting step as in the example shown in Fig. 14 is necessary here. It is noted that the fastening elements 40A', 40B' form the U-shaped fasteners 40', which are not visible here, but which are shown in Fig. 3 above. Thus, no cutting of the arcuate portions 30 is needed here. The so obtained reclosable fastener web 100 is then processed through a length cutting station comprising a cutting roller 228 and a counter roller 226 and then wound up to a storage or supply roll 230" of reclosable fastener web 100, wherein roller 231' facilitates reliable web transport and compression of the fastener web 100.

Fig. 16 shows in a schematic functional view a process of stretching a reclosable fastener web 100. As can be seen, the reclosable fastener web 100 made according to the processes as described herein and intermediately stored onto storage or supply roll 230, is being unwound and guided through nip rollers 310, 312, 310', 312' for further web processing. It is noted that the rollers 310, 312 guide the web with a dedicated speed which is slightly different compared to the speed of the web guided by rollers 310', 312'. The nip rollers help to control input and output speed of the fastener web. This includes the stretching of the reclosable fastener web 100 by subjecting the reclosable fastener web 100 to a stretching apparatus 300. The apparatus 300 may comprise heated rollers (e. g. 321 to 327) that soften backing layer 20' to improve stretching. The apparatus 300 may comprise rollers (e. g. 327 and 328) that run at different speed so that the fastener web gets elongated. The apparatus 300 may comprise further rollers (e. g. 329) to anneal the stretched backing layer. The obtained stretched reclosable fastener web 100" is then guided through the nip rollers 310' and 312'and might be cut in narrow lanes by slit roll 228 that cuts the web on a counter roll 226 and is finally wound up into a roll 230' for storage. This may help to reduce costs for making such reclosable fastener webs 100" as the roll length is increased. Such length orienting processes are used in combination with extruded films to form breathable backsheets. It is considered that this process could also be used for a breathable backsheet with fastening elements attached thereon.

Fig. 17 shows in a microscopy view a detail of a fiber provided in arcuate portions 30 during a preparation step of the precursor web 10 of the reclosable fastener web 100 according to the present disclosure. As can be seen, the fiber is shown prior to bonding to a backing layer 20 with its stem portion 28 and its arcuate portion 30 is arranged on a corrugation device 980. The corrugation device 980 comprises a plurality of ridges 982. In Fig. 17 two ridges 982 are shown. The ridges 982 have a repeat distance of 2,5 mm comprising a 1,7 mm groove followed by a 0,8 mm ridge. The depth of the groove is 1,5 mm. It is noted that in Fig. 17 only one fiber is exemplarily shown. The fiber is provided in a substantially sinusoidal wave structure for bonding to a backing layer. The precursor webs 10, 10' and the reclosable fastener webs 100, 100' shown in Figs. 18 to 24 have been made using the corrugation tool 980 as shown in Fig. 17.

Fig. 18 shows in a microscopy view the detail of precursor web 10. The picture shows the fiber as shown in Fig. 17 after bonding to a backing layer 20. As can be seen, the fiber comprising the stem portion 28 and the arcuate portion 30 has been bonded to the backing layer 20. Thereby the flat intermediate portions 26 were formed on the fiber. The fiber is bonded with the flat intermediate portion 26 to the backing layer 20 at bonding areas 22 which have been formed by the ultrasonic bonding process. Beside the bonding area 22, an intermediate space 24 is illustrated, where no bonding of the fiber is present. Similar to Fig. 17, it is noted that in Fig. 18 only one fiber is exemplarily shown.

Fig. 19 shows in a microscopy view the precursor web 10 of the reclosable fastener web 100 similar to an embodiment as shown in Fig. 18. It is noted that, contrary to Figs. 17 and 18, where only one fiber is exemplarily shown, Fig. 19 shows a plurality of fibers each having a stem portion 28 and an arcuate portion 30. The fibers in Fig. 19 are bonded to the backing layer 20, e. g. by ultrasonic bonding, wherein the bonding areas 22 have been formed at which the fibers with their flat intermediate portions 26 (not visible here, please see Fig. 18) formed by the bonding process are bonded to the backing layer 20. Several bonding areas 22 are visible in the example shown in Fig. 19 which are arranged as elongated lines repeatedly and in parallel to each other along the extension of the backing layer 20 perpendicular to the bonding areas 22. Between the bonding areas 22, intermediate spaces 24 indicated with 24 are present at the precursor web 10, which are not visible because of the plurality of arcuate portions 30 of the fibers bonded to the backing layer 20. The precursor web 10 is also illustrated in a schematic perspective view in Fig. 1 above. The precursor web 10 that is shown in Fig. 19 comprises a backing layer 20 and a corrugated fiber web formed by the arcuate portions 30 and the stem portions 28 and are attached to the backing layer 20. The backing layer is a 40 g/m² spunbond nonwoven as available under the trade name Pegatex S from company PFNonwovens, Czech Republic. The fiber layer has a basis weight of 30 g/m² and is made from 30 dtex staple fibers available under the trade name L10 from company Asota, Austria.

Fig. 20 shows in a microscopy view the precursor web 10' of the reclosable fastener web 100 representing a later process stage of the precursor web 10 as shown in Fig. 19. Different to the precursor web 10 as shown in Fig. 19, the precursor web 10' comprises fibers not having arcuate portions 30 any longer, but having stems 34 obtained by cutting the arcuate portions 30 as described in more detail above. The precursor web 10' also comprises bonding areas 22, which- similar to the precursor web 10 as shown in Fig. 19 - are arranged with line-like extension repeatedly and in parallel to each other along an extension of the backing layer 20 perpendicular to the extension of the bonding areas 22. Between the bonding areas 22, intermediate spaces 24 are arranged, wherein the intermediate portions 24 are not visible here because of the stems 34. The precursor web 10' is also illustrated in a schematic perspective view in Fig. 2 above. In Fig. 20 cutting was made to result in a stem height of 0,8 mm.

Fig. 21 shows in a microscopy view the U-shaped fasteners 40 of the reclosable fastener web 100 according to an embodiment of the present disclosure obtained from the precursor webs 10, 10' as shown in Figs. 19 and 20. The U-shaped fasteners 40 each comprise fastening elements 40A, 40B each having a stem 36 and an enlarged portion 38 at the distal end. The U-shaped fasteners 40 are bonded to the backing layer 20 of the reclosable fastener web 100 with their flat intermediate portions 26 (not visible here, please see Fig. 3) connecting the two fastening elements 40A, 40B to each other and bonding the U-shaped fasteners 40 to the backing layer 20 at bonding areas 22 (not visible here, please see Fig. 3). It is noted that the stems 36 as well as the enlarged portions 38 of the U-shaped fasteners 40 exhibit a substantially circular cross-section as the enlarged portions 38 have been obtained by heat treatment of the stems 36, i. e. showing substantially similar cross-sections.

Fig. 22 shows in a microscopy view the U-shaped fasteners 40 of the reclosable fastener web 100 according to an embodiment of the present disclosure similar to the embodiment as shown in Fig. 21. The difference here is that the fastening elements 40 comprise two stems 36 with enlarged portions 38 which do not have a substantially circular cross-section. Instead, the U-shaped fasteners 40 have been formed, as described in detail above, from fibers not having a circular cross-section, but having a stem 36 comprising four lobes 36A extending from the stem 36. Fig. 25 below illustrates in greater detail the stems 36 having four lobes 36A extending therefrom. Similar to the stems 36, the enlarged portions 38 in Fig. 22 also show lobes extending from the enlarged portions 38 as the enlarged portions 38 have been obtained by heat treatment of the stems 36, i. e. showing substantially similar cross-sections. In addition to Fig. 21, Fig. 22 also illustrates the flat intermediate portions 26 of the U-shaped fasteners 40 connecting the two fastening elements 40A, 40B to each other and bonding the U-shaped fasteners 40 to the backing layer 20 at bonding areas 22 having been formed by the bonding process, e. g. ultrasonic bonding as described in detail above. Fig. 22 also shows the intermediate spaces 24 arranged aside of the bonding areas 22. The reclosable fastener web 100 that is shown in Fig. 22 comprises a backing layer 20 and corrugated monofilament fibers attached to the backing layer in a spacing of 2 mm which have been transformed into the U-shaped fasteners 40 comprising the two fastening elements 40A, 40B each comprising a stem 36 and an enlarged portion 38 at the distal end thereof. The backing layer is a 40 g/m² spunbond nonwoven as available under the trade name Pegatex S from company PFNonwovens, Czech Republic. The monofilament has a tetralobal cross section and a diameter of 240 µm. Such filaments are available under the trade name MX215 from company Shakespeare, UK.

Fig. 23 shows in a top view the elastic ear 810 with the reclosable fastener web 100 thereon according to an embodiment of the present disclosure. The elastic ear 810 comprises an elastic core and one or two nonwoven-like skin layers (not illustrated here). The elastic ear 810 is attached to a hygiene article 820, for example an absorbent disposable article 820 such as a disposable diaper 820, only part of which is visible in Fig. 23. As can be seen, the reclosable fastener web 100 is attached in the form of a fastening tab 800 formed by a piece of the reclosable fastener 100 thereon to the side of the elastic ear 810 opposite to the side where the elastic ear is attached to the disposable diaper 820. The fastener web in Fig. 23 was made from the precursor 10 shown in Fig. 19. The softness of the reclosable fastener web 100 allows that U-shaped fasteners 40 are arranged on the entire surface of the tab 800, including the edges of the tab 800.

Fig. 24 shows in a top view a part of the hygiene article 820', for example a disposable diaper 820' according to an embodiment of the present disclosure. As can be seen, a fastening patch 800' with a piece of the reclosable fastener web 100 thereon is attached to the non-elastic area of the elastic ear 810' of the hygiene article 820'. As can be seen, the reclosable fastener web 100 is attached to the major surface of the elastic ear 810' at a distal end thereof which is also attached to the disposable diaper 820 at a proximal end thereof. Different to Fig. 23, the reclosable fastener web 100 is arranged as a patch 800' of the reclosable fastener web 100. The patch 800' does substantially not exceed the area of the elastic ear 810'. The construction of the tab 800 of Fig. 23 requires good strength to prevent tear of the tab. For the construction in Fig. 24, the strength is provided already by the elastic ear 810', so that the backing layer 20 of the reclosable fastener web 100 can be reduced. Fastening tab 800' has a 17 g/m² spunbond nonwoven as backing layer 20; the fiber web used to make the tab 800' is similar to the one shown and described in Fig. 19. This basis weight reduction results in an improved softness and reduced cost by reduced raw material consumption.

Fig. 25 shows in a schematic cross-sectional view the stem 36 of a U-shaped fastener 40 of the reclosable fastener web 100 according to an embodiment of the present disclosure. In the embodiment shown, the stem 36 has a non-circular cross-section having four lobes 36A. In Fig. 25, an inner stem diameter is illustrated by a dotted line with 36B, and an outer stem diameter is illustrated by a dotted line with 37. For good closure performance the ratio of outer diameter divided by inner diameter should be at least 3, more preferably at least 3,5. Fig. 25 can also be used to imagine a fiber cross section having only 3, 2, or 1 lobe extending from an inner stem diameter 36B. The non-circular cross section with lobes 36A extending from an inner stem diameter 36B also illustrates the reduced linear density versus a round fiber cross section of the outer diameter 37. This does reduce the bending stiffness of the fiber and makes corrugation easier. It also allows for higher hook density at a given basis weight of the fiber web.

Fig. 26 shows in a schematic side view a layer of the reclosable fastener web 100 according to an embodiment of the present disclosure. The reclosable fastener web 100 comprises a backing 20 to which U-shaped fasteners 40 with their two fastening elements 40A, 40B are attached. The U-shaped fasteners 40 and the fastening elements 40A, 40B, respectively, comprise a stem 36 and an enlarged portion 38 at the distal end of the stem 36. As can be seen, the fastening elements 40A, 40B of the U-shaped fasteners 40 exhibit an uncompressed thickness T, i. e. where the reclosable fastener web 100 has not yet undergone any compression, e. g. after the capping process.

Fig. 27 shows in a schematic side view several layers of the reclosable fastener web 100 according to an embodiment of the present disclosure after compression, e. g. as wound in a roll. In contrast to Fig. 26 showing the reclosable fastener web 100 before compression, the reclosable fastener web 100 in Fig. 27 has been compressed and wound up into a roll (not visible here) such that the thickness of the reclosable fastener web is changing from the uncompressed thickness T to the compressed thickness t as illustrated in Fig. 27. The fastening elements 40A, 40B and their stems 36, respectively, are bent and exhibit a curved shape as illustrated in Fig. 27, whereas in Fig. 26 the stems 36 of the fastening elements 40A, 40B of the U-shaped fasteners exhibit a rather straight shape as can be seen in Fig. 26. Such an arrangement may increase the effective roll length, i. e. more of the reclosable fastener web 100 can be stored on a storage or supply roll (not shown here) at a given outer dimension, i. e. diameter. This may increase the process efficiency as the supply roll needs to be changed at a lower frequency.

## Claims

1. A reclosable fastener web (100, 100') comprising
- a backing layer (20, 20') having a first and a second major surface,
- a plurality of U-shaped fasteners (40, 40') attached to the first major surface of the backing layer (20, 20'),
- wherein U-shaped fasteners (40, 40') each comprise two fastening elements (40A, 40B, 40A', 40B') each having a stem (36) with an enlarged portion (38) at the distal end and wherein the U-shaped fasteners (40, 40') further comprise a flat intermediate portion (26) between the two stems (36),
- wherein the stem (36) is a section of a fiber having an elongation at break of less than 400 %, preferably of less than 350 % and
- wherein the stem (36) has a linear density of less than 50 dtex.

2. A reclosable fastener web (100, 100') according to claim 1 comprising repeatedly arranged bonding areas (22, 22') at which the stems (36) are attached with their flat intermediate portions (26) to the first major surface of the backing layer (20, 20').

3. A reclosable fastener web (100, 100') according to claim 1 or 2, wherein the plurality of U-shaped fasteners (40, 40') protrude from the first major surface of the backing layer (20, 20') with random spacing along the bonding area (22, 22').

4. A reclosable fastener web (100, 100') according to any one of claims 1 to 3, wherein the reclosable fastener web (100, 100') is self-engaging.

5. A reclosable fastener web (100, 100') according to any one of the previous claims, wherein the stems (36) have a linear density of less than 30 dtex, preferably of less than 20 dtex, most preferred less than 15 dtex.

6. A reclosable fastener web (100, 100') according to any one of the previous claims, wherein the stems (36) have a non-circular cross section having at least one lobe (36A).

7. A reclosable fastener web (100, 100') according to any of claims 1 to 6 comprising a plurality of mechanical fastener tabs provided on the reclosable fastener web (100, 100') side-by-side in an endless form, wherein individual mechanical fastener tabs can be cut from the reclosable fastener web (100, 100').

8. A method of making a reclosable fastener web (100, 100') comprising the steps of
a. providing a fiber web (470, 570, 670, 770, 470', 570', 670', 770') comprising fibers with a linear density of less than 50 dtex; preferably of less than 30 dtex, most preferred of less than 20 dtex;
b. raising the fibers of the fiber web (470, 570, 670, 770, 470', 570', 670', 770') to provide fiber loops;
c. increase the number of fiber end sections by cutting fibers of the raised fiber loops to provide stems (36);
d. providing enlarged portions (38) at the distal end of the stems (36),
wherein the stems (36) preferably have a non-circular cross section with at least one lobe (36A).

9. The method according to claim 8, wherein the step of providing a fiber web (470, 570, 670, 770, 470', 570', 670', 770') comprises the steps of
- providing a plurality of fibers;
- making a fiber web (470, 570, 670, 770, 470', 570', 670', 770') from the provided fibers and
- optionally bonding the fiber web (470, 570, 670, 770, 470', 570', 670', 770').

10. The method according to any one of claims 8 or 9, wherein the step of forming raised fiber loops includes the steps of
- forming arcuate portions (30, 30') of the fiber web (470, 570, 670, 770, 470', 570', 670', 770');
- providing a backing layer (20, 20') and
- fixing the arcuate portions (30, 30') to the backing layer (20, 20') to create fiber loops.

11. The method according to claim 10, wherein, by fixing the arcuate portions (30, 30') onto the backing layer (20, 20'), flat intermediate portions (26) are formed on the fiber web (470, 570, 670, 770, 470', 570', 670', 770') at which the fiber loops are bonded to the backing layer (20, 20').

12. The method according to any one of claims 8 to 11 comprising the steps of
- compressing the reclosable fastener web (100, 100') from an initial thickness T to a compressed thickness t and
- winding the reclosable fastener web (100, 100') in the compressed state;
wherein the compressed thickness t is less than 0,8 T, preferably less than 0,6 T.

13. A fibrous hook and loop closure system comprising
- a loop component comprising a loop surface comprising loops which are sections of a fiber and which are configured and arranged for engaging with a hook component;
- a hook component comprising a reclosable fastener web (100, 100') according to any one of claims 1 to 7 comprising U-shaped fasteners (40, 40') for engaging with the loops of the loop component; wherein the U-shaped fasteners (40, 40') each comprising two fastening elements (40A, 40B, 40A', 40B') each having a stem (36) which is a section of a fiber with a linear density of less than 50 dtex and which comprise enlarged portion (38) at their distal ends,
- wherein the ratio of the linear density of the fibers from which the stems (36) are made divided by the linear density of the fibers from which the loops are made is less than 12, preferably less than 7 and
- wherein the U-shaped fasteners (40, 40') are made from fibers preferably having a non-circular cross section with at least one lobe (36A).

14. An elastic ear (810, 810') for a hygiene article (820, 820') with fastening properties comprising
- an elastic center layer,
- at least one nonwoven skin layer,
- a reclosable fastener web (100, 100') according to any of claims 1 to 7 attached to or attachable to the hygiene article-facing side of the elastic ear (810, 810'),
wherein the outer edge of the reclosable fastener web (100, 100') preferably does not exceed the outer edges of the elastic ear (810, 810').

15. A hygiene article (820, 820') comprising a reclosable fastener web (100, 100') according to any one of claims 1 to 7.
